# EUROPEAN PATENT APPLICATION

(11) **EP 4 763 980 A1**
(43) Date of publication of application: **24.06.2026**
(21) Application number: 25226361.1
(22) Date of filing: 22.12.2025
(51) Int. Cl.: C12N 9/00, C11D 3/386, C12P 21/02, C12N 9/28, C12N 9/54

(54) **SIDE PRODUCT INACTIVATION FOR ENZYMES IN BLEND**

(30) Priority: 23.12.2024 EP 24222834
(71) Applicant: BASF SE, 67056 Ludwigshafen am Rhein (DE)
(72) Inventor: Jenewein, Stefan, 67056 Ludwigshafen (DE); Riedele, Christian, 67056 Ludwigshafen am Rhein (DE); Mertsch, Alexander, 67056 Ludwigshafen am Rhein (DE); Kling-Kuebelbeck, Sonja, 67056 Ludwigshafen am Rhein (DE)
(74) Representative: Altmann Stößel Dick Patentanwälte PartG mbB

(57) **Abstract**

The present invention relates to a method for preparing an enzyme blend comprising the following steps: providing a fermentation-derived preparation of a first enzyme of interest; exposing said preparation of a first enzyme of interest to a temperature above the fermentation temperature; purifying the first enzyme from the fermentation-derived preparation; and adding at least one second enzyme of interest to the purified first enzyme; thereby obtaining an enzyme blend. Further, the present invention relates to an enzyme blend obtainable by or obtained by the method according to the invention.

## Description

### Technical Field

The present invention relates to a method for preparing an enzyme blend comprising the following steps: providing a fermentation-derived preparation of a first enzyme of interest; exposing said preparation of a first enzyme of interest to a temperature above the fermentation temperature; purifying the first enzyme from the fermentation-derived preparation; and adding at least one second enzyme of interest to the purified first enzyme; thereby obtaining an enzyme blend. Further, the present invention relates to an enzyme blend obtainable by or obtained by the method according to the invention.

### Background art

Enzymes are widely used in industrial and house hold applications and further find increasing use as sustainable ingredients reducing petro-chemistry based ingredients. Enzymes are biodegradable and can be catalytically active already at relatively low temperatures, which results in reduction of energy consumption. In detergent compositions, enzymes are widely used, for example, for improving cleaning efficiency and for reducing energy consumption during the cleaning process. In order to simplify the manufacturing of detergent compositions, enzymes are often prepared and marketed as enzyme blends. However, the use of enzymes in blends and detergents is hampered by the instability of these biomolecules, as they may degrade themselves, degrade each other and/or may be degraded by undesired host cell enzymatic activity.

### Problem to be solved

There is hence still a need for providing stable enzyme blends, in particular enzyme blends comprising detergent enzymes.

### Summary

This problem is addressed by a method and an enzyme blend with the features of the independent claims. Advantageous embodiments which might be realized in an isolated fashion or in any arbitrary combinations are listed in the dependent claims as well as throughout the specification.

The present invention hence relates to a method for preparing an enzyme blend. The method comprises the following steps which specifically may be performed in the given order or in a different order. Thus, a different order may also be possible. Further, two or more process steps may be performed simultaneously or partially simultaneously. Further, one or more than one or even all of the method steps may be performed once or more than once or even repeatedly or continuously. The method may further comprise additional method steps which are not listed. The method comprises the following steps:
a) providing a fermentation-derived preparation of a first enzyme of interest;
b) exposing said preparation of a first enzyme of interest to a temperature above the fermentation temperature; c) purifying the first enzyme from the fermentation-derived preparation; and d) adding at least one second enzyme of interest to the purified first enzyme; thereby e) obtaining an enzyme blend.

The inventors have surprisingly found that exposing a first enzyme preparation, for example an amylase preparation, to a temperature above the fermentation temperature prior to mixing it with a second enzyme preparation, such as a mannanase preparation, has the advantage of stabilizing the enzyme blend. More surprisingly, it was observed that particularly the second enzyme of interest can be stabilized. Without wishing to be bound by theory, it is thought that the temperature exposition contributes to inactivating undesired host cell enzymatic activity, in particular hydrolytic activity from host cell enzymes such as host cell lipases or host cell proteases. Heating of an enzyme blend comprising a heat labile enzyme is not a viable solution as said enzyme may be degraded during this heat treatment. Further advantages of the method according to the invention be higher throughput rates during purification and increase in purity of the resulting enzyme preparation.

As used herein, the terms "have", "comprise" or "include" or any arbitrary grammatical variations thereof are used in a non-exclusive way. Thus, these terms may both refer to a situation in which, besides the feature introduced by these terms, no further features are present in the entity described in this context and to a situation in which one or more further features are present. As an example, the expressions "A has B", "A comprises B" and "A includes B" may both refer to a situation in which, besides B, no other element is present in A (i.e. a situation in which A solely and exclusively consists of B) and to a situation in which, besides B, one or more further elements are present in entity A, such as element C, elements C and D or even further elements.

Further, it shall be noted that the terms "at least one", "one or more" or similar expressions indicating that a feature or element may be present once or more than once typically are used only once when introducing the respective feature or element. In most cases, when referring to the respective feature or element, the expressions "at least one" or "one or more" are not repeated, non-withstanding the fact that the respective feature or element may be present once or more than once.

Further, as used herein, the terms "preferably", "more preferably", "particularly", "more particularly", "specifically", "more specifically" or similar terms are used in conjunction with optional features, without restricting alternative possibilities. Thus, features introduced by these terms are optional features and are not intended to restrict the scope of the claims in any way. The invention may, as the skilled person will recognize, be performed by using alternative features. Similarly, features introduced by "in an embodiment of the invention" or similar expressions are intended to be optional features, without any restriction regarding alternative embodiments of the invention, without any restrictions regarding the scope of the invention and without any restriction regarding the possibility of combining the features introduced in such way with other optional or non-optional features of the invention.

As used herein, the singular forms of "a" and "an" include the respective plurals unless the context clearly dictates otherwise. In the context of the present invention, the terms "about" and "approximately" refer to an interval of accuracy that a person skilled in the art will understand to still ensure the technical effect of the feature in question. Typically, the terms indicate a deviation from the indicated numerical value of ±20 %, preferably ±15 %, more preferably ±10 %, and even more preferably ±5 %.

Furthermore, the terms "first", "second", "third" or "(a)", "(b)", "(c)", "(d)" etc. and the like are used herein for distinguishing between similar elements and not necessarily for describing a sequential or chronological order. It is to be understood that the terms so used are interchangeable under appropriate circumstances and that the embodiments of the invention described herein, in particular the method steps, may be operated in other sequences than described or illustrated herein. In case the terms "first", "second", "third" or "(a)", "(b)", "(c)", "(d)", "i", "ii" etc. relate to steps of a method or use or assay there is no time or time interval coherence between the steps, i.e. the steps may be carried out simultaneously or there may be time intervals of seconds, minutes, hours, days, weeks, months or even years between such steps, unless otherwise indicated in the application as set forth herein above or below.

It is to be understood that the term "comprising" is not limiting. For the purposes of the present invention the term "consisting of is considered to be a preferred embodiment of the term "comprising". If hereinafter a group is defined to comprise at least a certain number of embodiments, this is meant to also encompass a group which preferably consists of these embodiments only.

The method for preparing an enzyme blend according to the invention comprises a step of
a) providing a fermentation-derived preparation of a first enzyme of interest.

The term "providing a fermentation-derived preparation of an enzyme of interest" refers to any way of making available said fermentation-derived preparation. The term specifically may refer, without limitation, to allocating said enzyme preparation for processing in the method according to the invention, by ways of placing said preparation into a suitable vessel or container to allow for performing the method steps foreseen by the invention. Said step of providing the fermentation-derived preparation may be automated or may be performed manually. Means and methods for manual or automated provision of a fermentation-derived preparation are known in the art.

A "fermentation-derived preparation of an enzyme of interest" in line with the present invention is understood as any preparation derivable from a fermentation process comprising the enzyme of interest in any amount, typically in a solubilized state, crystalline or precipitated state, in particular in a solubilized state. More typically, said preparation is an aqueous solution. Still more typically, a fermentation-derived preparation of the enzyme of interest may be obtained by processing of a fermentation medium comprising a host cell producing the enzyme of interest. Said preparation may further refer to a supernatant obtained by a solid-liquid separation of the fermentation broth at the end of the fermentation process comprising the enzyme of interest.

More typically, said fermentation-derived preparation of the enzyme of interest may be obtained by a solid-liquid separation, for example filtration, crossflow filtration and/or centrifugation at the end of a fermentation process, for example at the time of harvest. The solid liquid separation may include a step of solubilization, dilution, stabilization and/or other processes. Even more typically, the fermentation-derived preparation of the enzyme of interest is a permeate, a filtrate, concentrate or a supernatant, typically obtained during solid liquid-separation after fermentation. As outlined elsewhere herein, the fermentation-derived preparation may preferably be derived from a bacterial fermentation, more preferably from a bacterial fermentation comprising Bacillus host cells producing the enzyme of interest.

Typically, the fermentation-derived preparation is obtained by a solid-liquid separation step followed by further processing steps such as microfiltration, anion exchange, chromatography, separation of remaining solids, ultrafiltration, evaporation, centrifugation, spray-drying, granulation, freeze-drying, crystallization, stabilization and the like, typically microfiltration.

More typically, the fermentation-derived preparation has been processed in a concentration step, involving ultra-filtration or evaporation or crystallization or other means for concentration known in the art.

The term "enzyme of interest" as referred to herein typically refers to any enzyme, full length or fragment providing the desired enzymatic activity thereof, which is intended to be produced in a fermentation process; in particular it refers to the full length mature enzyme. The term "enzyme of interest", thus, encompasses enzymes, or functional fragments thereof as well as fusion proteins, labeled enzymes and the like. The term "enzyme of interest" in particular relates to the first enzyme of interest and/or the second enzyme of interest and/or any further enzyme of interest according to the invention. Typically, the enzyme of interest is produced in a fermentation process; more typically, it is produced by a host cell carrying a gene encoding for the enzyme of interest; even more typically, the host cell contains an expression construct or vector for a gene encoding an enzyme of interest as described in further detail elsewhere herein. The enzyme of interest preferably has a desired enzymatic activity.

"Enzymatic activity" typically means the catalytic effect exerted by an enzyme, expressed as units per milligram of enzyme (specific activity) or molecules of substrate transformed per minute per molecule of enzyme (molecular activity). Enzymatic activity can be specified by the enzymes actual function, e.g. proteases exerting proteolytic activity by catalyzing hydrolytic cleavage of peptide bonds, lipases exerting lipolytic activity by hydrolytic cleavage of ester bonds, etc. Enzymatic activity may be desired, in particular if relating to the activity of the enzyme of interest. Further, enzymatic activity may be undesired, such as an enzymatic activity of aco-purified host cell enzyme. Undesired enzymatic activity may comprise an undesired activity of an esterase, for example undesired hydrolytic activity or undesired lipolytic activity, particularly undesired proteolytic activity or undesired lipolytic activity. Said undesired esterolytic activity may typically arise from co-purified host cell esterases, in particular undesired proteolytic activity may arise from co-purified host cell proteases, while said undesired lipolytic activity may typically arise form co-purified hos cell lipases. Enzymatic activity can be measured by means and methods known in the art. A method for determining an enzymatic activity according to the art, mannanase activity, is exemplified in example 3 below.

Preferably, the enzyme of interest is classified as an oxidoreductase (EC 1), a transferase (EC 2), a hydrolase (EC 3), a lyase (EC 4), an isomerase (EC 5), or a ligase (EC 6) (EC-numbering according to Enzyme Nomenclature, Recommendations (1992) of the Nomenclature Committee of the International Union of Biochemistry and Molecular Biology including its supplements published 1993-1999). In a preferred embodiment, the enzyme of interest is an enzyme suitable to be used in detergents.

More preferably, the enzyme is a hydrolase (EC 3), preferably, a glycosidase (EC 3.2) or a peptidase (EC 3.4). Especially, preferred enzymes are enzymes selected from the group consisting of an amylase (in particular an alpha-amylase (EC 3.2.1.1)), a cellulase (EC 3.2.1.4), a lactase (EC 3.2.1.108), a mannanase (EC 3.2.1.25), a lipase (EC 3.1.1.3), a phytase (EC 3.1.3.8), a nuclease (EC 3.1.11 to EC 3.1.31), and a protease (EC 3.4); in particular amylase, alpha-amylase, glucoamylase, pullulanase, protease, metalloprotease, peptidase, lipase, cutinase, acyl transferase, cellulase, endoglucanase, glucosidase, cellubiohydrolase, xylanase, xyloglucantransfer-ase, xylosidase, mannanase, phytase, phosphatase, xylose isomerase, glucoase isomerase, lactase, acetolactate decarboxylase, pectinase, pectin methylesterase, polygalacturonidase, lyase, pectate lyase, arabinase, arabinofuranosidase, galactanase, a laccase, peroxidase and an asparaginase.

Enzymes of particular interest may further be selected from: protease, oxidoreductase, transferase, hydrolase, lyase, isomerase, ligase, aminopeptidase, amylase, asparaginase, carbohydrase, carboxypeptidase, catalase, cellulase, chitinase, cutinase, cyclodextrin glycosyltransferase, deoxyribonuclease, esterase, alpha-galactosidase, betagalactosidase, glucoamylase, alpha-glucosidase, beta-glucosidase, hyaluronic acid synthase, invertase, laccase, lipase, mannosidase, mutanase, oxidase, a pectinolytic enzyme, peroxidase, phytase, polyphenoloxidase, ribonuclease, transglutaminase, dispersin, and xylanase.

More particularly, the enzyme of interest may be selected from the group consisting of: amylase, protease, lipase, lactase, mannanase, phytase, xylanase, phosphatase, glucoamylase, nuclease, and cellulase, even more particularly selected from the group consisting of: amylase, protease, lipase, mannanase, phytase, xylanase, phosphatase, glucoamylase, nuclease, and cellulase, more preferably the enzyme of interest is selected from amylase, and protease; even more preferably the enzyme of interest is a protease, for example a serine protease such as subtilisin protease; even more preferably the enzyme of interest is an amylase, such as alpha-amylase.

In line with the present invention, the first enzyme of interest may typically be an enzyme selected from the group consisting of amylase, mannanase, xylanase, phytase, and cellulase. Preferably the first enzyme of interest is an amylase; more preferably the first enzyme of interest is alpha-amylase.

Preferred amylases include those of bacterial or fungal origin (EC 3.2.1.1 and 3.2.1.2, respectively), more preferably, amylases of bacterial or fungal origin which are typically heat resistant. Particularly preferred are amylases derived from Bacillus species. Amylases of Bacillus species are considered advantageous as they typically offer excellent heat resistance as specified elsewhere herein. Further preferred are alpha-amylases (EC 3.2.1.1).

Particularly suitable amylases include amylases originating from Bacillus licheniformis; more particularly alpha-amylase having SEQ ID NO:2 as described in WO 95/10603 and variants thereof. Suitable variants may carry one or more amino acid substitutions and are described in WO 95/10603. WO 95/10603 describes variants comprising one or more substitutions in the following positions: 15, 23, 105, 106, 124, 128, 133, 154, 156, 178, 179, 181, 188, 190, 197, 201, 202, 207, 208, 209, 211, 243, 264, 304, 305, 391, 408, and 444 which have amylolytic activity and which may be particularly suitable in line with present invention. Further variants are de-scribed in WO 94/02597, WO 94/018314, WO 97/043424 and may typically include SEQ ID NO:4 of WO 99/019467. Amylases further may be derived from B. stearothermophilus. A typical amylase may be an amylase having SEQ ID NO:6 as disclosed in WO 02/10355 or an amylase with optionally having a C-terminal truncation over the wildtype sequence. Suitable variants of SEQ ID NO:6 include those comprising a deletion in positions 179 and/or 181 and/or 182 and/or a substitution in position 193. Amylases further maybe from Bacillus sp.707 having SEQ ID NO:6 as disclosed in WO 99/19467 and variants having a sequence identity of at least 95% thereto. Preferred variants of SEQ NO:6 are those having a substitution, a deletion or an insertion in one or more of the following positions: R181, G182, H183, G184, N195, I206, E212, E216 and K269. Amylases further may be from Bacillus halmapalus having SEQ ID NO:2 or SEQ ID NO:7 as described in WO 96/23872, also described herein as SP-722. Preferred variants are described in WO 97/3296, WO 99/194671 and WO 2013/001078. Amylases further may be from Bacillus sp. DSM 12649 having SEQ ID NO:4 as disclosed in WO 00/22103 and variants having a sequence identity of at least 95% thereto. Amylases further may be from Bacillus sp. A 7-7 (DSM 12368) having an amino acid sequence at least 95% identical to SEQ ID NO:2, in particular over the region of the amino acids 32 to 516 according to SEQ ID NO:2, as disclosed in WO 02/10356. Amylases further may be from Bacillus strain TS-23 having SEQ ID NO:2 as disclosed in WO 2009/061380 and variants thereof. Amylases further may be from Cy-tophaga sp. having SEQ ID NO:1 as disclosed in WO 2013/184577 and variants having a sequence identity of at least 95% thereto. Amylases further may be from Bacillus megaterium DSM 90 having SEQ ID NO:1 as disclosed in WO 2010/104675 and variants having an amino acid sequence at least 95% identical thereto. Amylases further may be from Bacillus sp. comprising amino acids 1 to 485 of SEQ ID NO:2 as described in WO 00/60060 and variants at least 95% thereto. Amylases further may be from Bacillus amyloliquefaciens or variants thereof, preferably selected from amylases according to SEQ ID NO: 3 as described in WO 2016/092009. Amylases may have SEQ ID NO: 12 as described in WO 2006/002643 or amylase variants thereof comprising the substitutions Y295F and M202LITV within said SEQ ID NO: 12. Amylases may have SEQ ID NO:6 as described in WO 2011/098531 or amylase variants comprising a substitution at one or more positions selected from the group consisting of 193 [G,A,S,T or M], 195 [F,W,Y,L,I or V], 197 [F,W,Y,L,I or V], 198 [Q or N], 200 [F,W,Y,L,I or V], 203 [F,W,Y,L,I or V], 206 [F,W,Y,N,L,I,V,H,Q,D or E], 210 [F,W,Y,L,I or V], 212 [F,W,Y,L,I or V], 213 [G,A,S,T or M] and 243 [F,W,Y,L, I or V] within said SEQ ID NO:6. Amylases may have SEQ ID NO:1 as described in WO 2013/001078 or amylase variants comprising an alteration at two or more (several) positions corresponding to positions G304, W140, W189, D134, E260, F262, W284, W347, W439, W469, G476, and G477 within said SEQ ID NO:1. Amylases may have SEQ ID NO:2 as described in WO 2013/001087 or may relate to amylase variants comprising a deletion of positions 181 and 182, or 182 and183, or 183 and 184, within said SEQ ID NO:2, optionally comprising one or two or more modifications in any of positions corresponding to W140, W159, W167, Q169, W189, E194, N260, F262, W284, F289, G304, G305, R320, W347, W439, W469, G476 and G477 within said SEQ ID NO:2. Amylases may be hybrid alpha-amylases from above mentioned amylases as for example as described in WO 2006/066594. Hybrid amylases may be according to WO 2014/183920 with A and B domains having at least 90% identity to SEQ ID NO:2 of WO 2014/183920 and a C domain having at least 90% identity to SEQ **ID** NO:6 of WO 2014/183920, wherein the hybrid amylase has amylolytic activity; preferably the hybrid alpha-amylase is at least 95% identical to SEQ ID NO: 23 of WO 2014/183920 and having amylolytic activity. Hybrid amylases may be according to WO 2014/183921 with A and B domains having at least 75% identity or more to SEQ ID NO: 2, SEQ ID NO: 15, SEQ **ID** NO: 20, SEQ ID NO: 23, SEQ ID NO: 29, SEQ ID NO: 26, SEQ ID NO: 32, and SEQ ID NO: 39 as disclosed in WO 2014/183921 and a C domain having at least 90% identity to SEQ ID NO: 6 of WO 2014/183921 , wherein the hybrid amylase has amylolytic activity; preferably, the hybrid alpha-amylase is at least 95% identical to SEQ ID NO: 30 as disclosed in WO 2014/183921 and having amylolytic activity; Hybrid amylases may be according to WO 2021/032881 comprising an A and B domain originating from the alpha amylase originating from Bacillus sp. A 7-7 (DSM 12368) and a C domain originating from the alpha-amylase from Bacillus cereus preferably, the A and B domain are at least 75% identical to the amino acid sequence of SEQ ID NO: 42 and a C domain is at least 75% identical to the amino acid sequence of SEQ ID NO: 44 - both sequences as disclosed in WO 2021/032881; more preferably, the hybrid amylase is at least 80% identical to SEQ ID NO:54 as disclosed in WO 2021/032881.

In one embodiment, the amylase is selected from commercially available amylases which include but are not limited to products sold under the trade names Duramyl^{™}, Ter- mamyl^{™}, Stainzyme^{™}, Stainzyme Plus^{™}, Natalase^{™}, Liquozyme X and BAN^{™}, Amplify^{™} Amplify Prime^{™} (from Novonesis A/S), and Rapidase^{™}, Purastar^{™}, Powerase^{™}, Effectenz^{™} (M100 from DuPont), Preferenz^{™} (S1000, S110 and F1000; from DuPont), Prima- Green^{™} (ALL; IFF), Optisize^{™} (IFF).

In line with the present invention, preferred amylases are amylases or amylase variants with a deletion of at least two amino acids positions in comparison to the parent alpha-amylase; said positions are selected from position 180, 181, 182, and 183 based on the parent alpha-amylase sequence given in SEQ ID NO:3 of the sequence listing disclosed herein.

Furthermore; in a preferred aspect, the amylase is a variant of a parent alpha-amylase comprising an amino acid alteration at one or more positions corresponding to amino acid positions selected from the group consisting 4, 6, 20, 25, 27, 28, 33, 38, 41, 70, 72, 73, 75, 81, 93, 94, 96, 115, 125, 126, 128, 134, 139, 160, 174, 175, 176, 179, 186, 195, 206, 210, 213, 250, 251, 253, 276, 291, 292, 299, 314, 318, 319, 323, 326, 338, 343, 352, 356, 359, 363, 364, 368, 372, 378, 379, 382, 385, 387, 388, 390, 393, 396, 398, 400, 405, 418, 434, 435, 447, 449, 450, 452, 454, 459, 460, 461, 465, 466 and 475 based on the parent alpha-amylase sequence given in SEQ ID NO:3; with following target mutations being particularly preferred (again with reference to SEQ ID NO:3 for numbering): 4Q, 6E, 6H, 20K, 25H, 25Y, 27I, 28V, 33M, 38V, 41C, 70H, 72C, 73C, 75L, 81L, 93Q, 94M, 96Q, 115T, 125K, 125R, 126D, 128C, 134F, 134Y, 139C, 160W, 174H, 175A, 176K, 179L, 186A, 186C, 186D, 186E, 186F, 186H, 186I, 186K, 186L, 186M, 186N, 186Q, 186R, 186S, 186V, 186W, 186Y, 195F, 206H, 206L, 206M, 206N, 206Y, 210C, 210D, 213C, 250I, 251E, 253C, 276R, 291T, 292C, 299S, 314Q, 318L, 319K, 323G, 326Y, 338S, 338T, 343W, 352C, 356V, 359W, 363M, 363S, 364T, 368F, 372C, 378E, 379A, 379R, 382Q, 385W, 387E, 387N, 387S, 388E, 388K, 388Y, 390F, 393Q, 396S, 398G, 400A, 400S, 405M, 418F, 434S, 434R, 435K, 447L, 449G, 450I, 452C, 454A, 459N, 460G, 461L, 461A, 465H, 466W and 475N. Particularly preferred amino acid exchanges are selected from the group consisting of: G4Q, N6E, N6H, D20K, N25H, N25Y, L27I, R28V, N33M, G38V, A41C, N70H, K72C, G73C, V75L, T81L, K93Q, S94M, G96Q, E115T, S125K, S125R, N126D, N128C, D134F, D134Y, A139C, Y160W, Q174H, N175A, R176K, K179L, G186A, G186C, G186D, G186E, G186F, G186H, G186I, G186K, G186L, G186M, G186N, G186Q, G186R, G186S, G186V, G186W, G186Y, N195F, I206H, I206L, I206M, I206N, I206Y, H210C, H210D, V213C, L2501, T251E, V253C, E276R, V291T, P292C, N299S, N314Q, V318L, Q319K, T323G, V326Y, E338S, E338T, F343W, A352C, T356V, R359W, Y363M, Y363S, P364T, Y368F, Y372C, G378E, V379A, V379R, M382Q, K385W, D387E, D387N, D387S, P388E, P388K, P388Y, L390F, R393Q, Y396S, Y398G, T400A, T400S, L405M, D418F, P434S, P434R, G435K, A447L, E449G, V450I, Y452C, I454A, T459N, N460G, T461L, T461A, N465H, K466W and S475N using SEQ ID NO: 3 for numbering. More preferred amino acid substitutions are realized in the amylase amino acid sequence of SEQ ID NO: 1 according to the sequence listing as disclosed herein.

Typically, the amylase in line with the present invention may comprise an amino acid sequence selected from the group consisting of SEQ ID NO:1 to SEQ ID NO:15. Further included are in particular variants of SEQ ID NO:1 to SEQ ID NO:15 and amylases comprising a sequence with at least 80 % of sequence identity to any of SEQ ID NO:1 to SEQ ID NO:15.

A particularly preferred amylase may comprise a sequence as specified in SEQ ID NO:1, SEQ ID NO:2, SEQ ID NO:4; SEQ ID: NO:5, SEQ ID: NO:6, SEQ ID: NO:7, SEQ ID: NO:8, SEQ ID: NO:9, SEQ ID: NO:10, SEQ ID: NO:11, SEQ ID: NO:14, or SEQ ID: NO: 15 or a variant thereof or having a sequence with at least 80 % of sequence identity thereto.

Typically, the mannanase in line with the present invention may comprise amino acid sequence selected from the group consisting of SEQ ID NO:16 to SEQ ID NO:18. Further included are in particular variants of SEQ ID NO:16 to SEQ ID NO:18 and amylases having a sequence with at least 80 % of sequence identity to any of SEQ ID NO:16 to SEQ ID NO:18.

A particularly preferred mannanase may have a sequence comprising the sequence as specified in SEQ ID NO:15, or SEQ ID NO:16, or a variant thereof or having a sequence with at least 80 % of sequence identity thereto.

In line with the present invention, preferably the first enzyme of interest is an amylase comprising an amino acid sequence comprising, preferably consisting of, any of SEQ ID NO:1 to SEQ ID NO:15 or variant thereof or an amylase comprising, or consisting of, a sequence with at least 80 % of sequence identity thereto; or the first enzyme of interest is a mannanase comprising, preferably consisting of a sequence selected from any of SEQ ID NO:16 to SEQ ID NO:18 or variant thereof or a mannanase comprising, or consisting of, a sequence with at least 80 % of sequence identity thereto. More preferably the first enzyme of interest is an amylase comprising an amino acid sequence selected from any of SEQ ID NO:1 to SEQ ID NO:15 or variant thereof or an amylase comprising a sequence with at least 80 % of sequence identity thereto, and wherein the second enzyme of interest is a mannanase comprising an amino acid sequence selected from any of SEQ ID NO:16 to SEQ ID NO:18 or variant thereof or a mannanase comprising a sequence with at least 80 % of sequence identity thereto; still more preferably, the first enzyme of interest is an amylase comprising a sequence according to SEQ ID NO:2 or variant thereof or an amylase comprising a sequence with at least 80 % of sequence identity to SEQ ID NO:2, and wherein the second enzyme of interest is a mannanase comprising a sequence according to SEQ ID NO:18 or variant thereof or a mannanase comprising a sequence with at least 80 % of sequence identity to SEQ ID NO:18.

Sequence identity usually is provided as "% sequence identity". To determine the percent-sequence-identity between two amino acid sequences in a first step a pairwise sequence alignment is generated between those two sequences, wherein the two sequences are aligned over their complete length (i.e., a pairwise global alignment). The alignment is, preferably, generated with a program implementing the Needleman and Wunsch algorithm (J. Mol. Biol. (1979) 48, p. 443-453), preferably by using the program "NEEDLE" (The European Molecular Biology Open Software Suite (EMBOSS)) with the programs default parameters (gap open=10.0, gap extend=0.5 and matrix=EBLOSUM62). The preferred alignment for the purpose of this invention is that alignment, from which the highest sequence identity can be determined. After aligning the two sequences, in a second step, an identity value shall be determined from the alignment. Therefore, according to the present invention the following calculation of percent-identity applies: x %-identity = (identical residues / length of the alignment region which is showing the respective sequence of this invention over its complete length) *100. Thus, sequence identity in relation to comparison of two amino acid sequences according to this embodiment is calculated by dividing the number of identical residues by the length of the alignment region which is showing the respective sequence of this invention over its complete length. This value is multiplied with 100 to give "% sequence identity".

In line with the invention, enzymes of interest may comprising an amino acid sequence which exhibit at least m %-identity to the sequences specified in SEQ ID NO:1 to SEQ ID NO:18 with m being an integer between 70 and 100, between 80 and 100 or between 90 and 100, preferably, 70, 75, 80, 85, 90, 91, 92, 93, 94, 95, 96, 97, 98 or 99 compared to the full length amino acid sequence, are expected to have essentially unchanged enzyme properties. Even more preferably, such enzymes or enzyme variants comprise an amino acid sequence which has at least 80%, at least 85%, at least 90%, at least 91%, at least 92%, at least 93%, at least 94% at least 95%, at least 96%, at least 97%, at least 98% or at least 99% sequence identity with the amino acid sequence shown in any one of SEQ ID NOs 1-18.

Enzyme variants comprising an amino acid sequence having at least one amino acid substitution, addition and/or deletion may, in particular, also comprise conservative mutations which appear to have a minimal effect on protein folding resulting in certain enzyme properties being substantially maintained when compared to the enzyme properties of the parent enzyme. For determination of %-similarity according to this invention the following applies, which is also in accordance with the BLOSUM62 matrix, which is one of the most used amino acids similarity matrix for database searching and sequence alignments: Amino acid A is similar to amino acids S; Amino acid D is similar to amino acids E; N; Amino acid E is similar to amino acids D; K; Q; Amino acid F is similar to amino acids W; Y; Amino acid H is similar to amino acids N; Y; Amino acid I is similar to amino acids L; M; V; Amino acid K is similar to amino acids E; Q; R; Amino acid L is similar to amino acids I; M; V; Amino acid M is similar to amino acids I; L; V; Amino acid N is similar to amino acids D; H; S; Amino acid Q is similar to amino acids E; K; R; Amino acid R is similar to amino acids K; Q; Amino acid S is similar to amino acids A; N; T; Amino acid T is similar to amino acids S; Amino acid V is similar to amino acids I; L; M; Amino acid W is similar to amino acids F; Y; Amino acid Y is similar to amino acids F; H; W.

Conservative amino acid substitutions may occur over the full length of the sequence of a polypeptide sequence of a functional protein such as an enzyme. Therefore, according to the present invention the following calculation of percent-similarity applies: m %-similarity = [ (identical residues + similar residues) / length of the alignment region which is showing the respective sequence of this invention over its complete length ] *100. Thus, sequence similarity in relation to comparison of two amino acid sequences herein is calculated by dividing the number of identical residues plus the number of similar residues by the length of the alignment region which is showing the respective sequence of this invention over its complete length. This value is multiplied with 100 to give "%-similarity". Especially, variant enzymes comprising conservative mutations which exhibit at least m %-similarity to the respective parent sequences with m being an integer between 70 and 100, between 80 and 100 or between 90 and 100, preferably, 70, 75, 80, 85, 90, 91, 92, 93, 94, 95, 96, 97, 98 or 99 compared to the full length polypeptide sequence, are expected to have essentially unchanged enzyme properties. Even more preferably, such variants comprise an amino acid sequence which has at least 80%, at least 85%, at least 90%, at least 91%, at least 92%, at least 93%, at least 94% at least 95%, at least 96%, at least 97%, at least 98% or at least 99% sequence similarity with the amino acid sequence shown in any one of SEQ ID NOs 1-18.

The second enzyme of interest may typically be selected from the group consisting of: protease, cellulase, mannanase, oxidoreductase, transferase, hydrolase, lyase, isomerase, ligase, aminopeptidase, asparaginase, carbohydrase, carboxypeptidase, catalase, chitinase, ctinase, cyclodextrin glycosyltransferase, deoxyribonuclease, endo-beta 1,3 glucanase, endo- beta-1,4-glucanase, xanthan endoglucanase, esterase, alpha-galactosidase, beta-galactosidase, glucoamylase, alpha-glucosidase, beta-glucosidase, glycosyl hydrolase, hyaluronic acid synthase, invertase, laccase, lipase, mannosidase, mutanase, oxidase, a pectinolytic enzyme, peroxidase, phytase, polyphenoloxidase, pullulanase, ribonuclease, transglutaminase, dispersin, xylanase, preferably selected from protease, mannanase, pectinase, lipase, cellulase including anti greying cellulase and carecellulase; more typically selected from the group consisting of: protease, mannanase, lipase, cellulase; even more typically the second enzyme of interest is a mannanase.

Preferably, the enzyme of interest is at least partially secreted by the host cells into the fermentation medium, or is expressed intracellularly by the host cells and only unintentionally released from the host cells into the fermentation medium, for example by lysis of the host cells. The term "fermentation broth" typically refers to the fermentation medium comprising a host cell producing the enzyme of interest; more typically the fermentation medium comprising a host cell producing the enzyme of interest and the enzyme of interest.

A "host cell" as used herein is a cell that supports the production of the enzyme of interest, typically in a fermentation process. More typically, the host cell is recombinant, e.g. it preferably contains a heterologous polynucleotide also referred to as "expression construct" which is introduced by man by gene technology and with regard to a polynucleotide includes all those constructions brought about by man by gene technology / recombinant DNA techniques known in the art. In other words; according to the invention a host cell refer to a cell which serves as a host for an expression construct or vector for a gene encoding an enzyme of interest. Said expression construct may be a naturally occurring expression construct, a recombinantly introduced expression construct or a naturally occurring expression construct which has been genetically modified in the bacterial cell.

The term "recombinant" is known to the person of skill in the art. In particular, "recombinant" (or transgenic) with regard to a cell or an organism means that the cell or organism contains a heterologous polynucleotide also referred to as "expression construct" which is introduced by man by gene technology and with regard to a polynucleotide includes all those constructions brought about by man by gene technology / recombinant DNA techniques known in the art.

The term "heterologous" (or exogenous or foreign or recombinant or non-native) polypeptide or protein is defined herein as a polypeptide that is not native to the host cell, a polypeptide or protein native to the host cell in which structural modifications, e.g., deletions, substitutions, and/or insertions, have been made by recombinant DNA techniques to alter the native polypeptide or protein, or a polypeptide/protein native to the host cell whose expression is quantitatively altered or whose expression is directed from a genomic location different from the native host cell as a result of manipulation of the DNA of the host cell by recombinant DNA techniques, or whose expression is quantitatively altered as a result of manipulation of the regulatory elements of the polynucleotide by recombinant DNA techniques e.g., a stronger promoter; or a polynucleotide native to the host cell, but integrated not within its natural genetic environment as a result of genetic manipulation by recombinant DNA techniques. The term "heterologously expressed" hence may typically refer to an enzyme of interest being expressed in a host cell from a heterologous expression construct. More typically, the host cell comprises the heterologous expression construct as a result of genetic manipulation by recombinant DNA techniques. These techniques are known in the art.

With respect to two or more polynucleotide sequences or two or more amino acid sequences, the term "heterologous" is used to characterize that the two or more polynucleotide sequences or two or more amino acid sequences are naturally not occurring in the specific combination with each other.

The term "nucleic acid" or "nucleic acid molecule" as used herein, includes reference to a deoxyribonucleotide (DNA) or ribonucleotide (RNA) polymer, i.e. a polynucleotide, in either single-or double-stranded form, and unless otherwise limited, encompasses known analogues having the essential nature of natural nucleotides in that they hybridize to single-stranded nucleic acids in a manner similar to naturally occurring nucleotides (e.g., peptide nucleic acids). A polynucleotide can be full-length or a sub-sequence of a native or heterologous structural or regulatory gene. Unless otherwise indicated, the term includes reference to the specified sequence as well as the complementary sequence thereof. Thus, DNAs or RNAs with backbones modified for stability or for other reasons are "polynucleotides" as that term is intended herein. Moreover, DNAs or RNAs comprising unusual bases, such as inosine, or modified bases, such as tritylated bases, to name just two examples, are "polynucleotides" as the term is used herein. It will be appreciated that a great variety of modifications have been made to DNA and RNA that serve many useful purposes known to those of skill in the art. The term "polynucleotide" as it is employed herein embraces such chemically, enzymatically or metabolically modified forms of polynucleotides, as well as the chemical forms of DNA and RNA characteristic of viruses and cells, including among other things, simple and complex cells. Every nucleic acid sequence herein that encodes a polypeptide or protein such as the enzyme of interest as defined herein also, by reference to the genetic code, describes every possible silent variation of the nucleic acid. The term "conservatively modified variants" applies to both amino acid and nucleic acid sequences. With respect to particular nucleic acid sequences, the term "conservatively modified variants" refers to those nucleic acids which encode identical or conservatively modified variants of the amino acid sequences due to the degeneracy of the genetic code. The term "degeneracy of the genetic code" refers to the fact that a large number of functionally identical nucleic acids encode any given protein. For instance, the codons GCA, GCC, GCG and GCU all encode the amino acid alanine. Thus, at every position where an alanine is specified by a codon, the codon can be altered to any of the corresponding codons described without altering the encoded polypeptide. Such nucleic acid variations are "silent variations" and represent one species of conservatively modified variation.

In line with the present invention, the host cell may express the enzyme of interest from an expression construct encoding an enzyme of interest. Typically, the host cell comprises an expression construct comprising at least a gene encoding for the enzyme of interest under the control of a promoter. More typically, the expression construct may be an arrangement of a gene of interest and expression control sequences and/or further elements including e.g. a secretory signal as specified elsewhere herein. In particular, the expression construct may comprise a secretory signal as specified elsewhere herein, which may specifically lead to secretion of the enzyme of interest into the fermentation medium. The said gene of interest, expression control sequences and/or further elements, or part thereof, may be native to, i.e., endogenously present in the genome of the host cell. Moreover, the term "expression construct" may also be understood as encompassing native expression constructs which have been genetically manipulated, e.g., by genomic editing and/or mutagenesis technologies.

A "promoter" as referred to herein is a nucleotide sequence located upstream of a gene on the same strand as the gene that enables transcription of said gene. A promoter may be used as an expression control sequence in accordance with the present invention. The activity of a promoter (also referred to as promoter activity) is understood herein as the capacity of the promoter to enable and initiate transcription of said gene, in other words it is understood as the capacity of the promoter to drive gene expression. The promoter is followed by the transcription start site of the gene. The promoter is recognized by an RNA polymerase, typically, together with the required transcription factors, which initiate transcription. A functional fragment or functional variant of a promoter is a nucleotide sequence which is recognizable by RNA polymerase and is capable of initiating transcription. Functional fragments or functional variants of promoters are also encompassed as a promoter in the sense of the present invention.

Promoters may for example be inducer-dependent promoters the activity of which depend on an activating signal molecule, i.e., the presence of an inducer molecule, or may be inducer-independent promoters, i.e. promoters that do not depend on the presence of an inducer molecule added to the fermentation medium and that are either constitutively active or can be increased in activity regardless of the presence of an inducer molecule that is added to the fermentation medium.

Preferably, the promoter is selected from the group consisting of the promoter sequences of the aprE promoter (a native promoter from the gene encoding the Bacillus subtilisin Carlsberg protease), amyQ promoter from Bacillus amyloliquefaciens, amyL promoter and variants thereof from Bacillus licheniformis (preferably as de-scribed in US5698415), bacteriophage SPO1 promoter, such as the promoter PE4, PE5, or P15 (preferably as described in WO2015118126 or in Stewart, C. R., Gaslightwala, I., Hinata, K., Krolikowski, K. A., Needleman, D. S., Peng, A. S., Peterman, M. A., Tobias, A., and Wei, P. 1998, Genes and regulatory sites of the "host-takeover module" in the terminal redundancy of Bacillus subtilis bacteriophage SPO1. Virology 246(2), 329-340), cryllIA promoter from Bacillus thuringiensis (preferably as described in WO9425612 or in Agaisse, H. and Lereclus, D. 1994. Structural and functional analysis of the promoter region involved in full expression of the crylllA toxin gene of Bacillus thuringiensis. Mol. Microbiol. 13(1). 97-107.), and combinations thereof, and active fragments or variants thereof.

Preferably, the promoter sequences can be combined with 5'-UTR sequences native or heterologous to the host cell, as described herein. Preferably, the promoter is an inducer-independent promoter. More preferably, the promoter is selected from the group consisting of: an veg promoter, lepA promoter, serA promoter, ymdA promoter, fba promoter, aprE promoter, amyQ promoter, amyL promoter, bacteriophage SPO1 promoter, crylllA promoter, combinations thereof, and active fragments or variants thereof. Even more preferably, the promoter sequence is selected from the group consisting of aprE promoter, amyL promoter, veg promoter, bacteriophage SPO1 promoter, and crylllA promoter, and combinations thereof, or active fragments or variants thereof. Still even more preferably, the promoter is selected from the group consisting of: an aprE promoter, SPO1 promoter, such as PE4, PE5, or P15 (preferably as described in WO15118126), tandem promoter comprising the promoter sequences amyl and amyQ (preferably as described in WO9943835), and triple promoter comprising the promoter sequences amyL, amyQ, and cryllla (preferably as described in WO2005098016). Most preferably, the promoter is an aprE promoter, preferably, an aprE promoter from Bacillus amyloliquefaciens, Bacillus clausii, Bacillus haloduans, Bacillus lentus, Bacillus licheniformis, Bacillus pumilus, Bacillus subtilis, or Bacillus velezensis, more preferably from Bacillus licheniformis, Bacillus pumilus or Bacillus subtilis, most preferably, from Bacillus licheniformis.

Typically, in line with the present invention, the host cell may be selected from the group consisting of: a bacterial cell, a yeast cell, a fungal cell, an algal cell or a cyanobacterial cell, a non-human animal cell or a non-human mammalian cell, and a plant cell. More typically, the host cell can be selected from any one of the following organisms:

### Bacteria:

The bacterial host cell can, for example, be selected from the group consisting of the genera Escherichia, Klebsiella, Helicobacter, Bacillus, Lactobacillus, Streptococcus, Amycolatopsis, Rhodobacter, Pseudomonas, Paracoccus, Lactococcus, Ensifer or Pantoea.

Useful gram positive bacterial host cells include, but are not limited to, a Bacillus cell, e.g., Bacillus alkalophius, Bacillus amyloliquefaciens, Bacillus brevis, Bacillus circulans, Bacillus clausii, Bacillus coagulans, Bacillus firmus, Bacillus Jautus, Bacillus lentus, Bacillus licheniformis, Bacillus megaterium, Bacillus pumilus, Bacillus stearothermophilus, Bacillus subtilis, and Bacillus thuringiensis. Most preferred, the prokaryote is a Bacillus cell, preferably, a Bacillus cell of Bacillus subtilis, Bacillus pumilus, Bacillus licheniformis, or Bacillus lentus.

Some other preferred bacteria include strains of the order Actinomycetales, preferably, Streptomyces, preferably Streptomyces spheroides (ATTC 23965), Streptomyces thermoviolaceus (IFO 12382), Streptomyces lividans or Streptomyces murinus or Streptoverticillum verticillium ssp. verticillium. Other preferred bacteria include Rhodobacter sphaeroides, Rhodomonas pal-ustri, Streptococcus lactis. Further preferred bacteria include strains belonging to Myxococcus, e.g., M. virescens.

Useful gram negative bacteria include: Escherichia, Pseudomonas, Rhodobacter, Paracoccus, Ensifer or Pantoea species: Preferred gram negative bacteria are Escherichia coli, Pseudomonas sp., preferably, Pseudomonas purrocinia (ATCC 15958) or Pseudomonas fluorescens (NRRL B-11) or Pseudomonas denitrificans, Rhodobacter capsulatus or Rhodobacter sphaeroides, Paracoccus carotinifaciens, Paracoccus zeaxanthinifaciens, Pantoea ananatis, or Sinorhizobium meliloti also known as Ensifer meliloti.

Further, the host cell may be a fungal cell. "Fungi" as used herein includes the phyla Ascomycota, Basidiomycota, Chytridiomycota, and Zygomycota as well as the Oomycota and Deuteromycotina and all mitosporic fungi. Representative groups of Ascomycota include, e.g., Neurospora, Eupenicillium (=Penicillium), Emericella (=Aspergillus), Eurotium (=Aspergillus), Thermothelomyces and the true yeasts listed below. Examples of Basidiomycota include mushrooms, rusts, and smuts. Representative groups of Chytridiomycota include, e.g., Allomyces, Blastocladiella, Coelomomyces, and aquatic fungi. Representative groups of Oomycota include, e.g. Saprolegniomycetous aquatic fungi (water molds) such as Achlya. Examples of mitosporic fungi include Aspergillus, Penicillium, Candida, and Alternaria. Representative groups of Zygomycota include, e.g., Rhizopus and Mucor.

Some preferred fungi include strains belonging to the subdivision Deuteromycotina, class Hyphomycetes, e.g., Fusarium, Humicola, Tricoderma, Myrothecium, Verticillum, Arthromyces, Caldariomyces, Ulocladium, Embellisia, Cladosporium or Dreschlera, in particular Fusarium oxysporum (DSM 2672), Humicola insolens, Trichoderma resii, Myrothecium verrucana (IFO 6113), Verticillum alboatrum, Verticillum dahlie, Arthromyces ramosus (FERM P-7754), Caldariomyces fumago, Ulocladium chartarum, Embellisia alli Dreschlera halodes or Thermothelomyces thermophiles (ATCC 42464) also referred to as Myceliophthora thermophila. Other preferred fungi include strains belonging to the subdivision Basidiomycotina, class Basidiomycetes, e.g. Coprinus, Phanerochaete, Coriolus or Trametes, in particular Coprinus cinereus f. microsporus (IFO 8371), Coprinus macrorhizus, Phanerochaete chrysosporium (e.g. NA-12) or Trametes (previously called Polyporus), e.g. T. versicolor (e.g. PR4 28-A).

Further preferred fungi include strains belonging to the subdivision Zygomycotina, class Mycoraceae, e.g. Rhizopus or Mucor, in particular Mucor hiemalis.

The fungal host cell may be a yeast cell. Yeast as used herein includes ascosporogenous yeast (Endomycetales), basidiosporogenous yeast, and yeast belonging to the Fungi Imperfecti (Blastomycetes). The ascosporogenous yeasts are divided into the families Spermophthoraceae and Saccharomycetaceae. The latter is comprised of four subfamilies, Schizosaccharomycoideae (e.g., genus Schizosaccharomyces), Nadsonioideae, Lipomycoideae, and Saccharomycoideae (e.g. genera Kluyveromyces, Pichia, and Saccharomyces). The basidiosporogenous yeasts include the genera Leucosporidim, Rhodosporidium, Sporidiobolus, Filobasidium, and Filobasidiella. Yeasts belonging to the Fungi Imperfecti are divided into two families, Sporobolomycetaceae (e.g., genera Sporobolomyces and Bullera) and Cryptococcaceae (e.g. genus Candida).

### Eukaryotes:

Eukaryotic host cells further include, without limitation, a non-human animal cell, a non-human mammal cell, an avian cell, reptilian cell, insect cell or a plant cell.

Still more preferably, said host cell is a microbial cell, such as a fungal cell, an archaea cell, an algae cell, a protozoa cell or a bacterial cell; more preferably a fungal or a bacterial cell. Bacterial host cells are preferred.

Bacterial host cells may in principle be selected from the group of bacteria consisting of Bacillus, Streptomyces, Escherichia, Buttiauxella and Pseudomonas. In the method of the invention, the host cell is preferably a cell of a Bacillus species.

According to the method of the invention, preferably, the fermentation is a bacterial fermentation process, more preferably the method comprises producing the enzyme of interest in a Bacillus host cell. Hence, typically, the fermentation-derived preparation is derived from a bacterial fermentation, more typically from a bacterial fermentation comprising Bacillus host cells producing the enzyme of interest.

As mentioned herein, the host cell in the method according to the invention may be a Bacillus cell. The Bacillus cell may be a cell from any member of the bacterial genus Bacillus, preferably a host cell of Bacillus licheniformis, Bacillus subtilis, Bacillus alkalophilus, Bacillus amyloliquefaciens, Bacillus brevis, Bacillus circulans, Bacillus clausii, Bacillus coagulans, Bacillus firmus, Bacillus jautus, Bacillus lentus, Bacillus megaterium, Bacillus pumilus, Bacillus stearothermophilus, Bacillus thuringiensis or Bacillus velezensis. More preferably, the Bacillus host cell is a Bacillus licheniformis, Bacillus pumilus, or Bacillus subtilis cell, even more preferred Bacillus licheniformis or Bacillus subtilis cell, most preferably, Bacillus licheniformis host cell. Particular preferably, the Bacillus licheniformis is selected from the group consisting of Bacillus licheniformis as deposited under American Type Culture Collection number ATCC 14580, ATCC 31972, ATCC 53926, ATCC 53757, ATCC 55768, and under DSMZ number (German Collection of Microorganisms and Cell Cultures GmbH) DSM 13, DSM 394, DSM 641, DSM 1913, DSM 11259, and DSM 26543.

Typically, the host cell belongs to the species Bacillus licheniformis, such as a host cell of the Bacillus licheniformis strain as deposited under American Type Culture Collection number ATCC 14580 (which is the same as DSM 13, see Veith et al. "The complete genome sequence of Bacillus licheniformis DSM 13, an organism with great industrial potential." J. Mol. Microbiol. Biotechnol. (2004) 7:204-211). Alternatively, the host cell may be a host cell of Bacillus licheniformis strain ATCC 53926. Alternatively, the host cell may be a host cell of Bacillus licheniformis strain ATCC 31972. Alternatively, the host cell may be a host cell of Bacillus licheniformis strain ATCC 53757. Alternatively, the host cell may be a host cell of Bacillus licheniformis strain ATCC 53926. Alternatively, the host cell may be a host cell of Bacillus licheniformis strain ATCC 55768. Alternatively, the host cell may be a host cell of Bacillus licheniformis strain DSM 394. Alternatively, the host cell may be a host cell of Bacillus licheniformis strain DSM 641. Alternatively, the host cell may be a host cell of Bacillus licheniformis strain DSM 1913. Alternatively, the host cell may be a host cell of Bacillus licheniformis strain DSM 11259. Alternatively, the host cell may be a host cell of Bacillus licheniformis strain DSM 26543.

According to the present invention, the fermentation preferably is a bacterial fermentation, more preferably comprising producing the enzyme of interest in a Bacillus host cell; even more preferably, the Bacillus host cell is a cell of Bacillus amyloliquefaciens, Bacillus clausii, Bacillus haloduans, Bacillus lentus, Bacillus licheniformis, Bacillus pumilus, Bacillus subtilis, or Bacillus velezensis, preferably Bacillus licheniformis.

A "fermentation process" as understood herein, typically comprises the cultivation of host cells, in particular the bacterial cells, more particularly the Bacillus cells, in a suitable fermentation medium, also referred to as "cultivation medium". "Cultivation of the cells" or "growth of the cells" is not understood to be limited to an exponential growth phase, but can also include the physiological state of the cells at the be-ginning of growth after inoculation and during a stationary phase. Typically, the cultivation may take place at a fermentation temperature suitable for supporting growth of the cells. More typically, the fermentation temperature is within the range of 20 °C to 40 °C, in particular in the range of 28°C to 38 °. The fermentation process may usually take place in a fermenter, preferably in a fermenter with a volume scale which is at least 1 m³.

An industrially relevant fermentation process encompasses a fermentation process on a volume scale which is 1-500 m³ with regard to the nominal fermenter size, preferably 5-500 m³, more preferably 10-500 m³, even more preferably 25-500 m³, most preferably 50-500 m³. In other words, an industrially relevant fermentation process encompasses a fermentation process on a volume scale which is at least 1000 L with regard to the nominal fermenter size, preferably at least 5,000 L, more preferably at least 10,000 L, even more preferably at least 25,000 L, still even more preferably at least 50,000 L and most preferably 50,000-500,000 L.

An "enzyme blend" as used herein refers to a mixture of at least two different enzymes of interest, in particular to preparations of at least two different enzymes of interest. Typically to a mixture comprising a first enzyme of interest and a second enzyme of interest. More typically, the first enzyme of interest is a heat-resistant enzyme, typically selected from a group of heat-resistant enzymes as specified elsewhere herein. Still more typically, the second enzyme of interest is selected from the group of enzymes as specified elsewhere herein, including heat labile enzymes. Typically the first enzyme and/or the second enzyme are selected from the class of detergent enzymes. One or more enzyme/s may be added as a third or further enzyme of interest. "Heat resistance" of an enzyme of interest, such as a detergent enzyme, is understood as a high degree of enzymatic activity after exposing the enzyme to heat, in particular after exposing the enzyme to a temperature above the fermentation temperature as defined elsewhere herein. Heat resistance of an enzyme is in particular understood as the overall enzymatic activity of the enzyme being reduced by at most 20%, preferably by at most 10%, more preferably by at most 5% of the overall enzymatic activity; e.g. the activity of the enzyme before heat exposure or before exposure to a temperature above the fermentation temperature. In other words, heat resistance of an enzyme is in particular understood as the enzymatic activity of the enzyme remaining at at least 80 %, at at least 90 % or at at least 95 %, after exposing the enzyme to heat, in particular after exposing the enzyme to a temperature above the fermentation temperature as compared to the activity of an un-exposed enzyme. A heat-resistant detergent enzyme in line with the invention, typically comprises a heat-resistant enzyme selected from the group consisting of: amylase, mannanase, xylanase, phytase, and cellulase. Heat resistance may already be an attribute of a wild-type enzyme and/or it may be engineered by means of protein engineering. An enzyme may more particularly be considered heat-resistant, if more than 90 % of its activity, specifically of its enzymatic activity prior to exposure to heat, e.g. exposure to a temperature above the fermentation temperature, is retained after exposure to a temperature above 60 °C, 70 °C, 80° or even above 90 °C for 30 min. Conditions during the temperature exposure are typically at the enzyme's optimal pH and in an aqueous buffer or water, more typically without added protein denaturants.

Typically, a third enzyme of interest may be selected from the group consisting of: protease, cellulase, mannanase, oxidoreductase, transferase, hydrolase, lyase, isomerase, ligase, aminopeptidase, asparaginase, carbohydrase, carboxypeptidase, catalase, chitinase, ctinase, cyclodextrin glycosyltransferase, deoxyribonuclease, endo-beta 1,3 glucanase, endo- beta-1,4-glucanase, xanthan endoglucanase, esterase, alpha-galactosidase, beta-galactosidase, glucoamylase, alpha-glucosidase, beta-glucosidase, glycosyl hydrolase, hyaluronic acid synthase, invertase, laccase, lipase, mannosidase, mutanase, oxidase, a pectinolytic enzyme, peroxidase, phytase, polyphenoloxidase, pullulanase, ribonuclease, transglutaminase, dispersin, xylanase, preferably selected from protease, mannanase, pectinase, lipase, cellulase including anti greying cellulase and carecellulase more typically selected from cellulase or lipase.

A preferred enzyme blend in line with the invention comprises amylase as a first enzyme of interest, and mannanase or protease as a second enzyme of interest, optionally a cellulase or lipase as a third enzyme of interest.

As outlined elsewhere herein said enzyme blend is obtained in step e). Hence, preferably, the enzyme blend obtained in step e) comprises at least amylase and mannanase; or at least amylase and protease.

Hence, the method according to the invention may comprise a step of adding at least one further enzyme that differs from the first and second enzyme of interest selected from the group consisting of: protease, mannanase, pectinase, lipase, cellulase; preferably selected from the group consisting of: protease, mannanase, lipase, cellulase.

Typically, the enzyme blend obtained in step e) comprises at least amylase and mannanase and wherein the enzyme blend comprises at least one or more further enzymes selected from the group consisting of: protease, cellulase, lipase.

The method according to the invention comprises a step b) of exposing said preparation of a first enzyme of interest to a temperature above the fermentation temperature. The term exposing said preparation to a temperature is understood herein as any suitable way of heating said preparation to a temperature above the fermentation temperature. Typically, the heating may be performed by using a heater as known in the art. In particular, heating may be performed in a fermentation vessel or fermenter, an incubator, a heating chamber, or using a boiler system, more particularly in a heatable fermentation vessel or fermenter. Preferably, said temperature above fermentation temperature is in the range of 50 °C to 85 °C; more preferably in the range of 50 °C to 80 °C; still more preferably in the range of 55 °C to 75°C, still more preferably in the range of 65 °C to 75 °C..

The exposing step is typically performed for a time sufficient to reduce undesired enzymatic activity, in particular hydrolytic activity, more particularly proteolytic activity. In particular, step b) of exposing said preparation of a first enzyme of interest to a temperature above the fermentation temperature is performed for a time sufficient to reduce undesired host cell enzyme activity. Hence, preferably the step of exposing the solution of said first enzyme reduces undesired enzymatic activity, for example undesired hydrolytic activity such as undesired activity of a protease or of an esterase, more preferably undesired hydrolytic activity such as proteolytic activity, or an undesired esterolytic or lipolytic activity.

More typically, said exposing step is performed for at least 1 min to several hours depending on the enzymatic activity to be reduced, and/or on the temperature stability of the enzyme of interest, typically depending on both. Further, the time of exposing may be done in parallel to another method step such as a solid-liquid separation step, in particular microfiltration. This is advantageous as the through-put rate, i.e. the flow through during microfiltration, may increase with the increase in temperature. Preferably, the exposing to a temperature above the fermentation temperature is performed for at least 1 minutes to at least 30 minutes.

Typically, step b) of exposing said preparation of a first enzyme of interest to a temperature above the fermentation temperature is performed after the fermentation process. More typically, step b) comprises a solid-liquid separation at a temperature above the fermentation temperature; even more typically, said solid-liquid separation comprises microfiltration. Means and methods for microfiltration are known to the person of skill in the art. Means and methods for exposing the first enzyme of interest to a temperature above the fermentation temperature are known in the art and typically include heating in a fermenter, or in a flow heater. Coarse filters may be used for removing particular material prior to step b).

In a preferred embodiment according to the invention, exposing said preparation of a first enzyme of interest to a temperature above the fermentation temperature may be performed simultaneously to a microfiltration step.

Typically, by said step b) of exposing said preparation of a first enzyme of interest to a temperature above the fermentation temperature a temperature-exposed first enzyme of interest may be obtained. It is understood herein, that said the step of exposing said first enzyme of interest with a temperature above the fermentation temperature is advantageous as it may surprisingly contribute to stabilizing an enzyme blend comprising said temperature-exposed first enzyme of interest and at least a second enzyme of interest. Said stabilization may surprisingly increase in particular the stability of the second enzyme of interest.

The method according to the invention comprises a step c) of purifying the first enzyme from the fermentation-derived preparation. By purifying the enzyme of interest, said enzyme is separated from other compounds or cells present in the fermentation-derived preparation. Means and methods for purifying an enzyme of interest from a fermentation derived preparation are known in the art. The purifying step of the fermentation-derived preparation of the first enzyme of interest may comprise at least one of the following: solid-liquid separation, clarification, separation of remaining solids, ultrafiltration, evaporation, centrifugation, spray drying, granulation, freeze-drying, stabilization, ion ex-change chromatography, salting out, adsorption and the like. Step b) of exposing the first enzyme of interest to a temperature above the fermentation temperature and step c) of purifying the first enzyme from the fermentation-derived preparation may be performed simultaneously or in any order such as step b) followed by step c) or vice versa. Purifying may involve adding of a stabilizing system as described elsewhere herein, in particular adding of an inorganic salt as calcium chloride.

Moreover, the method according to the invention comprises a step d) of adding at least one second enzyme of interest to the purified first enzyme. Typically, the second enzyme is added in solubilized form, for example in an aqueous solution, or in dry form, for example as a powder. The second enzyme may be a purified enzyme from a cell free preparation. It may typically be a cell-free preparation with a minimal enzyme concentration such as 1 % (w/w) active enzyme. Typically, the second enzyme may be a fermentation-derived preparation of an enzyme of interest as defined elsewhere herein. Adding may be performed by any suitable means and methods as known in the art for example by pouring, pipetting, microfluidics, supplying, pumping etc. and may be in particular be followed by a mixing step, for example stirring or agitating preferably using a suitable stirrer or agitating device as known in the art. Typically, the second enzyme has been purified from a fermentation broth by means and methods known in the art and as de-scribed elsewhere herein, for example as described with respect to the first enzyme. More typically, said second enzyme is a least purified by a solid-liquid separation step, said step may involve the use of clarifying agents such as polyaluminum salts. Even more typically said second enzyme has been purified prior to step d) of adding it to the purified first enzyme.

By adding the second enzyme of interest to the preparation comprising the first enzyme of interest, the enzyme blend may be obtained. Hence, the method according to the invention comprises a step e) of obtaining the enzyme blend. The obtained enzyme blend may then be processed further, for example by means of further purification steps; including concentrating the blend for example through evaporation; and/or packaging the blend into suitable containers.

The method according to the invention may comprise adjusting the pH of the fermentation-derived preparation to a value below 10.5; preferably to a value between 7 and 5. The pH value typically depends on the enzymes of interest present in the enzyme blend. The pH may more typically be adjusted prior to or after the step of exposing said preparation of a first enzyme of interest to a temperature above the fermentation temperature, typically thereafter.

The method may further comprise adding at least a stabilizing system, in particular an enzyme stabilizing system. Preferably, the enzyme stabilizing system comprises at least one compound selected from the group consisting of polyols (preferably, 1,3-propanediol, ethylene glycol, glycerol, 1,2-propanediol, or sorbitol), inorganic salts (preferably, CaCl₂, MgCl₂, or NaCl), short chain (preferably, C1-C3) carboxylic acids or salts thereof (preferably, formic acid, formate (preferably, sodium formate), acetic acid, acetate, or lactate), borate, boric acid, boronic acids (preferably, 4-formyl phenylboronic acid (4-FPBA)), peptide aldehydes (preferably, Benzyloxycarbony-VAL-H (Z-VAL-H or Cbz-VAL-H) or Benzyloxycarbony-GAY-H (Z-GAY-H or Cbz-GAY-H), peptide ace-tals, and peptide aldehyde hydrosulfite adducts. Preferably, the enzyme stabilizing system comprises a combination of at least two of the compounds selected from the group consisting of salts, polyols, and short chain carboxylic acids and preferably one or more of the compounds selected from the group consisting of borate, boric acid, boronic acids (preferably, 4-formyl phenylboronic acid (4-FPBA)), peptide aldehydes, peptide acetals, and peptide aldehyde hydrosulfite adducts. In a particularly preferred embodiment, the stabilizing system comprises a protease inhibitor, preferably selected from borate, boric acid, boronic acids (preferably, 4-FPBA), peptide aldehydes (preferably, peptide aldehydes like Z-VAL-H or Z-GAY-H), peptide acetals, and peptide aldehyde hydrosulfite adducts, preferably the protease inhibitor is a peptide aldehyde, preferably Z-VAL-H or Z-GAY-H. In one embodiment, the stabilizing system does not comprise a protease inhibitor. Preferably, the solution is boron-free. In particular, adding a stabilizing system may comprise treating the purified enzyme solution with an inorganic salt such as CaCl₂ as exemplified in examples 1 and 2 below. Preferably, the purified enzyme solution comprises a calcium salt, more preferably calcium chloride.

The present invention further relates to the use of a temperature-exposed first enzyme of interest for stabilizing an enzyme blend comprising the first enzyme of interest and at least a second enzyme of interest.

Moreover, the present invention contemplates an enzyme blend obtainable by or obtained by the method according to the invention. The enzyme blend is in particular characterized by its enhanced stability. This is demonstrated in the examples provided herein below.

Further, the present invention relates to a detergent composition comprising the enzyme blend according to the preceding embodiment, in particular a detergent composition suitable for dish washing, preferably automated dishwashing, or laundry.

The publications referred to herein and those references cited within those publications in their entireties are hereby incorporated by reference into this application in order to more fully describe the state of the art to which this invention pertains.

Summarizing and without excluding further possible embodiments, the following embodiments may be envisaged:
1. A method for preparing an enzyme blend comprising the following steps:
   a) providing a fermentation-derived preparation of a first enzyme of interest;
   b) exposing said preparation of a first enzyme of interest to a temperature above the fermentation temperature;
   c) purifying the first enzyme from the fermentation-derived preparation; and
   d) adding at least one second enzyme of interest to the purified first enzyme;
   e) thereby obtaining an enzyme blend.
2. The method according to the preceding embodiment, wherein the first enzyme of interest is a heat-resistant enzyme.
3. The method according to the preceding embodiment, wherein the first enzyme of interest is selected from the group consisting of amylase, mannanase, xylanase, phytase, and cellulase; preferably the first enzyme of interest is an amylase; more preferably the first enzyme of interest is alpha-amylase.
4. The method according to any of the preceding embodiments, wherein the second enzyme of interest is selected from the group consisting of: protease, cellulase, mannanase, oxidoreductase, transferase, hydrolase, lyase, isomerase, ligase, aminopeptidase, asparaginase, carbohydrase, carboxypeptidase, catalase, chitinase, cutinase, cyclodextrin glycosyltransferase, deoxyribonuclease, endo-beta 1,3 glucanase, endo- beta-1,4-glucanase, xanthan endoglucanase, esterase, alpha-galactosidase, beta-galactosidase, glucoamylase, alpha-glucosidase, beta-glucosidase, glycosyl hydrolase, hyaluronic acid synthase, invertase, laccase, lipase, mannosidase, mutanase, oxidase, a pectinolytic enzyme, peroxidase, phytase, polyphenoloxidase, pullulanase, ribonuclease, transglutaminase, dispersin, xylanase, preferably selected from protease, mannanase, pectinase, lipase, cellulase including anti greying cellulase and carecellulase; more preferably selected from the group consisting of: protease, mannanase, lipase, cellulase; even more preferably the second enzyme of interest is a mannanase.
5. The method according to any of the preceding embodiments, comprising a step of adding at least one further enzyme that differs from the first and second enzyme of interest selected from the group consisting of: protease, mannanase, pectinase, lipase, cellulase; preferably selected from the group consisting of: protease, mannanase, lipase, cellulase.
6. The method according to any of the preceding embodiments, wherein the fermentation temperature is in the range of 20°C to 40°C, preferably in the range of 28°C to 38 °C.
7. The method according to any of the preceding embodiments, wherein the temperature above the fermentation temperature in step b) is in the range of 50 °C to 85 °C; preferably in the range of 50 to 80 °C; more preferably in the range of 55°C to 75°C.
8. The method according to any of the preceding embodiments, wherein the enzyme blend obtained in step e) comprises at least amylase and mannanase; or at least amylase and protease.
9. The method according to any of the preceding embodiments, wherein the enzyme blend obtained in step e) comprises at least amylase and mannanase and wherein the enzyme blend comprises at least one or more further enzymes selected from the group consisting of: protease, cellulase, lipase.
10. The method according to any of the preceding embodiments, wherein the method further comprises adding at least a stabilizing system.
11. The method according to the preceding embodiment, wherein the stabilizing agent comprises at least one compound selected from the group consisting of polyols (preferably, 1,3-propanediol, ethylene glycol, glycerol, 1,2-propanediol, or sorbitol), inorganic salts (preferably, CaCl2, MgCl2, or NaCl), short chain (prefer-ably, C1-C3) carboxylic acids or salts thereof (preferably, formic acid, formate (preferably, sodium formate), acetic acid, acetate, or lactate), borate, boric acid, boronic acids (preferably, 4-formyl phenylboronic acid (4-FPBA)), peptide alde-hydes (preferably, Benzyloxycarbony-VAL-H (Z-VAL-H or Cbz-VAL-H) or Benzyloxycarbony-GAY-H (Z-GAY-H or Cbz-GAY-H), peptide acetals, and peptide aldehyde hydrosulfite adducts.
12. The method according to any one of the preceding embodiments, wherein step b) of exposing said preparation of a first enzyme of interest to a temperature above the fermentation temperature is performed after the fermentation process, or wherein step b) comprises a solid-liquid separation at a temperature above the fermentation temperature.
13. The method according to embodiment 12, wherein said solid-liquid separation comprises microfiltration.
14. The method according to embodiment 12 or 13, wherein step b) of exposing said preparation of a first enzyme of interest to a temperature above the fermentation temperature is performed simultaneously to a solid-liquid separation comprising microfiltration.
15. The method according to any one of the preceding embodiments, comprising adjusting the pH of the fermentation-derived preparation to a value below 10.5; preferably to a value between 7 and 5.
16. The method according to any one of the preceding embodiments, wherein the purifying step of the fermentation-derived preparation of the first enzyme of interest comprises at least one of the following: separation of remaining solids, ultrafiltration, evaporation, centrifugation, spray draying, granulation, freeze-drying, stabilization, ion exchange chromatography, and the like.
17. The method according to any one of the preceding embodiments, wherein the fermentation-derived preparation is derived from a bacterial fermentation, preferably from a bacterial fermentation comprising a Bacillus host cell producing the enzyme of interest.
18. The method according to the preceding embodiment, wherein the Bacillus host cell is a cell of Bacillus amyloliquefaciens, Bacillus clausii, Bacillus haloduans, Bacillus lentus, Bacillus licheniformis, Bacillus pumilus, Bacillus subtilis, or Bacillus velezensis, preferably Bacillus licheniformis.
19. The method according to any of the preceding embodiments, wherein step b) of exposing said preparation of a first enzyme of interest to a temperature above the fermentation temperature is performed for a time sufficient to reduce undesired host enzyme activity.
20. The method according to any of the preceding embodiments, wherein step b) of exposing said preparation of a first enzyme of interest to a temperature above the fermentation temperature is performed simultaneously with or after step c) of purifying the first enzyme from the fermentation-derived preparation.
21. The method according to any of the preceding embodiments, wherein the first enzyme of interest is an amylase comprising an amino acid sequence selected from any of SEQ ID NO:1 to SEQ ID NO:15 or variant thereof or an amylase comprising a sequence with at least 80 % of sequence identity thereto; or a mannanase comprising an amino acid sequence selected from any of SEQ ID NO:16 to SEQ ID NO:18 or variant thereof or a mannanase comprising a sequence with at least 80 % of sequence identity thereto.
23. The method according to any of the preceding embodiments, wherein the first enzyme of interest is an amylase comprising an amino acid sequence selected from any of SEQ ID NO:1 to SEQ ID NO:15 or variant thereof or an amylase comprising a sequence with at least 80 % of sequence identity thereto, and wherein the second enzyme of interest is a mannanase comprising an amino acid sequence selected from any of SEQ ID NO:16 to SEQ ID NO:18 or variant thereof or a mannanase comprising a sequence with at least 80 % of sequence identity thereto.
23. The method according to any of the preceding embodiments, wherein the first enzyme of interest is an amylase comprising a sequence according to SEQ ID NO:2 or variant thereof or an amylase comprising a sequence with at least 80 % of sequence identity thereto, and wherein the second enzyme of interest is a mannanase comprising a sequence according to SEQ ID NO:18 or variant thereof or a mannanase comprising a sequence with at least 80 % of sequence identity thereto.
24. Use of a temperature-exposed first enzyme of interest for stabilizing an enzyme blend comprising the first enzyme of interest and at least a second enzyme of interest.
25. An enzyme blend obtainable by or obtained by the method of any of the preceding embodiments.
26. A detergent composition comprising the enzyme blend according to the preceding embodiment, in particular a detergent composition suitable for dish washing, preferably automated dishwashing, or laundry.

The invention is not limited to one of the embodiments described above, but is modifiable in a great variety of ways. Those skilled in the art recognize that the embodiments according to the invention can easily be adapted without departing from the scope of the invention. Further characteristics, details and advantages of the invention follow from the wording of the claims, from the following description of practical examples and from the figures.

The content of all literature references cited in this patent application is hereby included by reference to the respective specific disclosure content and in its entirety.

The following examples serve to illustrate the invention. They must not be interpreted as limiting with regard to the scope of protection.

### SEQUENCES

SEQ ID NOs: 1-15 correspond to amylase sequences.
SEQ ID NOs: 16-18 correspond to mannanase sequences.

### FIGURES

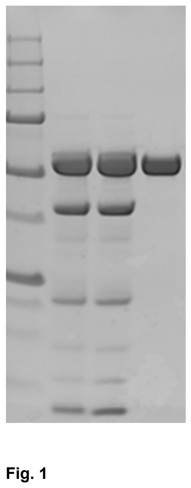

**Figure 1** shows an SDS-PAGE of a 70 °C treatment after fermentation. Lanes from left to right: Precision Plus Protein^{™} All Blue Prestained Protein Standard from Biorad; Sample 1: Supernatant of the end of fermentation of an amylase; Sample 2: Supernatant of the chemically treated fermentation broth containing amylase but no heat treatment ( = Incubation only at 30°C). Sample 3: Supernatant of the chemically and heat treated fermentation broth containing amylase (70 °C).

### EXAMPLES

### Example 1 Cultivation and processing of amylase and mannanase

### Amylase

*A Bacillus licheniformis* strain comprising a gene expressing an alpha amylase as shown in SEQ ID NO:2 was cultivated in a fermentation process using a chemically defined fermentation medium as described in the reference fermentation of example 1 from WO2024/028338 with the temperature set to 35 °C.

After the cultivation the fermentation broth was heated in the fermentation vessel to two different temperatures (as indicated in the Table 1 of Example 3: 30 °C and 70°C incubation temperature) for 90 min and cooled to 30 °C after the heat step. For further purification, the amylase containing samples were always supplemented, independent of the presence of a heating step, with following chemicals (given for 1 kg fermentation broth; under stirring conditions): 2 kg water and 9 g CaCl₂x2H₂O. The control sample was only treated with chemicals without prior heat treatment step. The pH was adjusted to pH 9.5 using NaOH. The soluble amylase was separated from the biomass using a Sartoflow Advanced (Sartorius Stedim Systems GmbH) device and Atech membrane modules (0.2 µm) and further concentrated using a Sartorius Sartoflow Advanced device PES membrane with 10 kDa pore size (Sartocon cassette, PES, 10 kDa; Sartorius Stedim Biotech GmbH). The amylase was formulated in 50% glycerol and the pH was adjusted to pH 6. The heat treatment did not cause an inactivation of the amylase by more than 10% of the overall activity.

### Mannanase

A *Bacillus licheniformis* strain comprising a gene expressing a mannanase such shown in SEQ ID NO 18, was cultivated in a fermentation process using a chemically defined fermentation medium as described in the reference fermentation of example 1 from WO2024/028338 with the temperature set to 35 °C.

After cultivation, the mannanase containing broth was supplemented with following chemicals (given for 1 kg of fermentation broth; under stirring conditions): 35 g of PAX-18 (polyaluminum chloride, Kemira). The pH was adjusted during addition of PAX-18 to pH 6. The mannanase was separated from the biomass using a Sartoflow Advanced (Sartorius Stedim Systems GmbH) device and Atech membrane modules (0.2 µm) and further concentrated using a Sartorius Sartoflow Advanced device PES membrane with 10 kDa pore size (Sartocon cassette, PES, 10 kDa; Sartorius Stedim Biotech GmbH). The mannanase was stored in 50% glycerol prior use at pH 6.

### Example 2 Analysis of heat treatment during amylases purification

To qualitatively assess the effect of the heat treatment during amylase processing analysis by SDS PAGE was performed using the following samples:
Sample 1: Supernatant at the end of fermentation.
Sample 2: Supernatant of the chemically treated fermentation broth (water and CaCl₂x2H₂O)
Sample 3: Supernatant of the heat treated fermentation broth

As sample buffer NuPAGE LDS sample buffer supplemented with 10 mM EDTA (final concentration) was used. Samples were adjusted to 2 mg/mL, mixed 1+1 with sample buffer and 10 µL were loaded on the PAGE. SDS PAGE was performed using precast gels (Invitrogen^{™} Nu-PAGE^{™} 4-12 %, Bis-Tris, 1,0 mm) and NuPAGE MES SDS running buffer. As marker the_Precision Plus Protein^{™} All Blue Prestained Protein Standard from Biorad was used.

In Figure 1 (heat treatment at 70°C) it is visible that the purity of the amylase sample treated at 70 °C is significantly higher than a sample that was only kept at 30°C.

### Example 3 Storage of Amylase and Mannanase

To test the effect of the heat treatment of the amylase on the stability of a secondary enzyme such as the mannanase, the following formulation was generated: 0.4% w/w amylase from example 1, 0.1% w/w mannanase from example 1, 50% glycerol,1% monophenyl glycol (pH adjusted to pH 6). A reference formulation, including the same components except without the addition of the amylase, was generated.

The formulations were stored at 45 °C for up to 28 days with samples taken at the following time points: Day 0, Day 7, Day 14, Day 21 and Day 28.

Residual mannanase activity of the samples taken at different time points was measured using AZCL-Galactomannan (CAROB) from Megazymes (I-AZGMA) as substrate. 100 µL of the enzyme containing solution (or a suitable dilution) was added to 900 µL 0,2 % AZCL-Galactomannan (CAROB) (dissolved in 100mM Tris buffer (pH8.6); supplemented with 0,1% Brij 35 and 0,1% Xanthan gum). After incubation at 40 °C for 30 minutes in a deep well plate the insoluble substrate was removed by centrifugation. 200 µL supernatant from reaction plate were carefully transferred to a fresh 96well plate and the absorbance was measured at 590 nm in a plate reader. Calibration was done using a suitable reference mannanase solution.

The residual mannanase activity was normalized in reference to the initial time point and is thus given in %. The results from the storage test in residual mannanase activity are given in table 1. The residual mannanase activity is significantly increased in formulations with amylase derived from process with a heat treatment at 70 °C.

**Table 1. Residual mannanase activity in amylase-mannanase blends over time. x indicates that the respective enzyme was added, or that the 70 °C heat treatment was conducted.**

| | | | | Residual mannanase activity [%] | | | | |
|---|---|---|---|---|---|---|---|---|
| Sample no | Mannanase 0.1% | Amylase 0.4% | Heat treatment of amylase containing broth | Day 0 | Day 7 | Day 14 | Day 21 | Day 28 |
| 1 | x | | | 100 | 89 | n.a. | 79 | 78 |
| 2 | x | x | x | 100 | 82 | 75 | n.a. | 67 |
| 3 | x | x | x | 100 | 90 | 82 | 79 | 74 |
| 4 | x | x | x | 100 | 97 | 93 | 84 | 77 |
| 5 | x | x | x | 100 | 96 | 78 | 70 | 57 |
| 6 | x | x | x | 100 | 96 | 88 | 78 | 71 |
| 7 | x | x | x | 100 | 93 | 80 | 77 | 73 |
| 8 | x | x | 30°C = no heat treatment | 100 | 60 | 41 | 29 | 23 |

Sample 1 is a reference sample for the activity of the second enzyme (mannanase) without the addition of the first enzyme (amylase). Sample 8 is a reference sample for the activity of the second enzyme with the addition of the first enzyme, but no heat treatment. Sample 2-7 are samples according the invention where heat treatment was pursued showing higher stability of the second enzyme over the reference sample 8.

## Claims

1. A method for preparing an enzyme blend comprising the following steps:
a) providing a fermentation-derived preparation of a first enzyme of interest;
b) exposing said preparation of a first enzyme of interest to a temperature above the fermentation temperature;
c) purifying the first enzyme from the fermentation-derived preparation;
d) adding at least one second enzyme of interest to the purified first enzyme; and
e) thereby obtaining an enzyme blend;
wherein sad first enzyme is a heat-resistant enzyme.

2. The method according to the preceding claim, wherein the first enzyme of interest is selected from the group consisting of amylase, mannanase, xylanase, phytase, and cellulase; preferably the first enzyme of interest is an amylase; more preferably the first enzyme of interest is alpha-amylase.

3. The method for according to any one the preceding claims, wherein the second enzyme of interest is selected from the group consisting of: protease, cellulase, mannanase, oxidoreductase, transferase, hydrolase, lyase, isomerase, ligase, aminopeptidase, asparaginase, carbohydrase, carboxypeptidase, catalase, chitinase, cutinase, cyclodextrin glycosyltransferase, deoxyribonuclease, endo-beta 1,3 glucanase, endo- beta-1,4-glucanase, xanthan endoglucanase, esterase, alpha-galactosidase, beta-galactosidase, glucoamylase, alpha-glucosidase, beta-glucosidase, glycosyl hydrolase, hyaluronic acid synthase, invertase, laccase, lipase, mannosidase, mutanase, oxidase, a pectinolytic enzyme, peroxidase, phytase, polyphenoloxidase, pullulanase, ribonuclease, transglutaminase, dispersin, xylanase, preferably selected from protease, mannanase, pectinase, lipase, cellulase including anti greying cellulase and carecellulase; more preferably selected from the group consisting of: protease, mannanase, lipase, cellulase; even more preferably the second enzyme of interest is a mannanase.

4. The method for according to any one the preceding claims, wherein the temperature above the fermentation temperature in step b) is in the range of 50 °C to 85 °C; preferably in the range of 50 to 80 °C; more preferably in the range of 55°C to 75°C.

5. The method for according to any one the preceding claims, wherein the method further comprises adding at least a stabilizing system.

6. The method according to any of the preceding claims, wherein the stabilizing system comprises at least one compound selected from the group consisting of polyols (preferably, 1,3-propanediol, ethylene glycol, glycerol, 1,2-propanediol, or sorbitol), inorganic salts (preferably, CaCl₂, MgCl₂, or NaCl), short chain (preferably, C1-C3) carboxylic acids or salts thereof (preferably, formic acid, formate (preferably, sodium formate), acetic acid, acetate, or lactate), borate, boric acid, boronic acids (preferably, 4-formyl phenylboronic acid (4-FPBA)), peptide alde-hydes (preferably, Benzyloxycarbony-VAL-H (Z-VAL-H or Cbz-VAL-H) or Benzyloxycarbony-GAY-H (Z-GAY-H or Cbz-GAY-H), peptide ace-tals, and peptide aldehyde hydrosulfite adducts.

7. The method according to any of the preceding claims, wherein step b) of exposing said preparation of a first enzyme of interest to a temperature above the fermentation temperature is performed after the fermentation process, or wherein step b) comprises a solid-liquid separation at a temperature above the fermentation temperature.

8. The method according to claim 7, wherein said solid-liquid separation comprises microfiltration.

9. The method according to claim 7 or claim 8, wherein step b) of exposing said preparation of a first enzyme of interest to a temperature above the fermentation temperature is performed simultaneously to a solid-liquid separation comprising microfiltration.

10. The method according to any of the preceding claims, wherein step b) of exposing said preparation of a first enzyme of interest to a temperature above the fermentation temperature is performed simultaneously to a solid-liquid separation comprising microfiltration

11. The method according to any of the preceding claims, wherein the fermentation-derived preparation is derived from a bacterial fermentation, preferably from a bacterial fermentation comprising a Bacillus host cell producing the enzyme of interest.

12. The method according to any of the preceding claims, wherein step b) of exposing said preparation of a first enzyme of interest to a temperature above the fermentation temperature is performed for a time sufficient to reduce undesired host enzyme activity

13. Use of a temperature-exposed first enzyme of interest for stabilizing an enzyme blend comprising the first enzyme of interest and at least a second enzyme of interest.

14. An enzyme blend obtainable by or obtained by the method of any of the preceding claims.

15. A detergent composition comprising the enzyme blend according to the preceding claim, in particular a detergent composition suitable for dish washing, preferably automated dishwashing, or laundry.
